# EUROPEAN PATENT SPECIFICATION

(11) **EP 4 314 906 B1**
(45) Date of publication and mention of the grant of the patent: **04.03.2026**
(21) Application number: 22778256.2
(22) Date of filing: 30.03.2022
(51) Int. Cl.: G01V 9/00, G06V 20/13, G01V 20/00, G06V 10/774, G06V 10/776, G06N 20/20

(54) **SYSTEMS AND METHODS FOR SOIL MAPPING**
SYSTEME UND VERFAHREN ZUR BODENKARTIERUNG
SYSTÈMES ET PROCÉDÉS DE CARTOGRAPHIE DU SOL

(30) Priority: 31.03.2021 US 202163169035 P
(43) Date of publication of application: 07.02.2024
(73) Proprietor: Terramera, Inc., Vancouver BC V5Y 1K3 (CA)
(72) Inventor: GOOD, Travis Godwin, Vancouver, British Columbia V5Y 1K3 (CA); ROTHWELL, Austin Caulfield, Vancouver, British Columbia V5Y 1K3 (CA)
(74) Representative: FRKelly
(86) International application number: PCT/CA2022/050475
(87) International publication number: WO 2022/204809

(56) References cited:
- CA-A1- 3 120 370
- CA-A1- 3 120 370
- US-A1- 2020 163 272
- US-A1- 2020 163 272
- TAGHIZADEH-MEHRJARDI RUHOLLAH ET AL: "Bio-Inspired Hybridization of Artificial Neural Networks: An Application for Mapping the Spatial Distribution of Soil Texture Fractions", REMOTE SENSING, vol. 13, no. 5, 8 March 2021 (2021-03-08), CH, pages 1025, XP055976038, ISSN: 2072-4292, DOI: 10.3390/rs13051025
- STUMPF FELIX ET AL: "Uncertainty-guided sampling to improve digital soil maps", CATENA, ELSEVIER, AMSTERDAM, NL, vol. 153, 31 January 2017 (2017-01-31), pages 30 - 38, XP029953638, ISSN: 0341-8162, DOI: 10.1016/J.CATENA.2017.01.033
- RINGROSE-VOASE A J ET AL: "Four Pillars of digital land resource mapping to address information and capacity shortages in developing countries", GEODERMA, ELSEVIER, AMSTERDAM, NL, vol. 352, 28 October 2017 (2017-10-28), pages 299 - 313, XP085772722, ISSN: 0016-7061, [retrieved on 20171028], DOI: 10.1016/J.GEODERMA.2017.10.014
- WADOUX ALEXANDRE M J-C ED - ADAMCHUK VIACHESLAV I ET AL: "Using deep learning for multivariate mapping of soil with quantified uncertainty", GEODERMA, vol. 351, 22 May 2019 (2019-05-22), pages 59 - 70, XP085706375, ISSN: 0016-7061, DOI: 10.1016/J.GEODERMA.2019.05.012
- TAGHIZADEH-MEHRJARDI RUHOLLAH, EMADI MOSTAFA, CHERATI ALI, HEUNG BRANDON, MOSAVI AMIR, SCHOLTEN THOMAS: "Bio-Inspired Hybridization of Artificial Neural Networks: An Application for Mapping the Spatial Distribution of Soil Texture Fractions", REMOTE SENSING, MOLECULAR DIVERSITY PRESERVATION INTERNATIONAL (MDPI), CH, vol. 13, no. 5, 8 March 2021 (2021-03-08), CH , pages 1025, XP055976038, ISSN: 2072-4292, DOI: 10.3390/rs13051025

## Description

### Technical Field

The present disclosure relates generally to systems and methods for soil mapping, and particularly to systems and methods for determining sampling locations for measuring soil characteristics.

### Background

Soil maps provide information about soil properties over an area of interest and are used for purposes such as land evaluation, agricultural planning, environmental protection assessments, etc. Developing a soil map generally involves characterizing soil properties across the area of interest based on a collection of soil measurements (e.g. physical soil samples rigorously characterized via laboratory analysis) and/or environmental data (e.g. elevation, climate, satellite imagery, and so on). The former tend to provide more accurate measures of soil properties but can be challenging to obtain in sufficient quantity and quality across a large area of interest.

Although soil maps can be derived directly from high-quality, quantitative soil measurements, they are more often created using digital soil mapping techniques in which environmental variables are correlated with a small number of soil measurements. The environmental variables may then be used to predict soil characteristics across the area of interest relatively more expediently and/or at lower cost than using solely high-quality, quantitative soil measurements, although generally at the expense of accuracy of the resulting soil map; for instance, soil maps are often inaccurate in areas dissimilar to those where quantitative soil data was collected. Models for correlating soil characteristics and environmental variables include kriging, geographically weighted regression, random forest, and neural networks.

Some soil mapping techniques use specific sampling approaches to determine locations from which to collect a small number of soil measurements to form a basis for generating predictions from environmental data. Sampling approaches include random sampling, uniform sampling, stratified sampling, conditioned Latin hypercube sampling, and orthogonal sampling. A suitable sampling approach can, in appropriate circumstances, reduce the number of high-quality, quantitative soil measurements required to achieve a certain level of performance and/or improve accuracy of the soil map relative to less suitable sampling measurements.

There is a general desire for systems and methods for determining sampling locations for measuring soil characteristics to generate soil maps efficiently and/or accurately.

STUMPF FELIX ET AL. "Uncertainty-guided sampling to improve digital soil maps" CATENA, ELSEVIER, AMSTERDAM, NL, vol. 153, 31 January 2017 (2017-01-31) describes Digital Soil Mapping (DSM) to estimate spatial variability of soil properties. The document describes two approaches for purposive sampling design: The first approach uses a Random Forest model calibration, where legacy samples are evaluated, and additional samples are placed in highly uncertain regions. The second approach involves replacing existing legacy samples with new ones in the same highly uncertain regions.

The foregoing examples of the related art and limitations related thereto are intended to be illustrative and not exclusive. Other limitations of the related art will become apparent to those of skill in the art upon a reading of the specification and a study of the drawings.

### Summary

The following embodiments and aspects thereof are described and illustrated in conjunction with systems, tools and methods which are meant to be exemplary and illustrative, not limiting in scope. In various embodiments, one or more of the above-described problems have been reduced or eliminated, while other embodiments are directed to other improvements.

The present invention provides a method for training a soil mapping model according to claim 1 and a computer system according to claim 14.

One aspect of the invention provides systems and methods for training a soil mapping model. The system comprises one or more processors and a memory storing instructions which cause the one or more processors to perform operations comprising the method. The method is performed by a processor and comprises: training a set of initial parameters for a soil mapping model relating one or more soil characteristics to one or more covariates over an area of interest based on an initial training dataset; determining an uncertainty map for the soil mapping model over at least a portion of the area of interest based on the initial parameters, the uncertainty map mapping each of a plurality of locations in the area of interest to a measure of uncertainty in at least one of the one or more soil characteristics; determining one or more sampling locations in the area of interest based on the uncertainty map; receiving one or more sample measurements corresponding to the one or more sampling locations; and refining the set of initial parameters of the soil mapping model based on the one or more sample measurements to generate a first set of refined parameters for the soil mapping model.

In some embodiments, the method comprises mapping at least one location in the area of interest to at least one predicted value of at least one of the one or more soil characteristics, the at least one predicted value based on at least one of: the first set of refined parameters and a further set of refined parameters based on the first set of refined parameters for the soil mapping model.

In some embodiments, the method comprises iteratively generating one or more further sets of refined parameters, each set of refined parameters generated by: determining a further uncertainty map for the soil mapping model based on at least one of the first set of refined parameters and the one or more further sets of refined parameters; determining one or more further sampling locations in the area of interest based on the further uncertainty map; receiving one or more further sample measurements corresponding to the one or more further sampling locations; and refining at least one of the first set of refined parameters and at least one further sets of refined parameters based on the one or more sample measurements to generate a further set of refined parameters for the soil mapping model.

In some embodiments, the method comprises mapping at least one location in the area of interest to at least one predicted value of at least one of the one or more soil characteristics, the at least one predicted value based on at least one of the further sets of refined parameters for the soil mapping model.

In some embodiments, iteratively generating the one or more further sets of refined parameters comprises determining a measure of model uncertainty based on at least one of the uncertainty maps; and halting generation of further refined parameters based on the measure of model uncertainty. In some embodiments, the measure of model uncertainty comprises an average of measures of uncertainty for the plurality of locations in the area of interest. In some embodiments, halting generation of further refined parameters based on the measure of model uncertainty comprises determining that the measure of model uncertainty is less than a threshold uncertainty value.

In some embodiments the plurality of locations mapped by the uncertainty map comprises a plurality of regions in the area of interest; and determining the one or more sampling locations comprises at least one of: random sampling, uniform sampling, stratified sampling, and conditioned Latin hypercube sampling, and orthogonal sampling in the at least one region.

In some embodiments, the method determining the one or more sampling locations comprises determining, for each one of the plurality of regions, a measure of aggregate uncertainty for the region; and selecting the at least one region based on the measures of aggregate uncertainty.

According to the invention, determining one or more sampling locations in the area of interest comprises selecting a first one of a plurality of candidate sampling locations, the first candidate sampling location being mapped to a measure of uncertainty by the uncertainty map at least as great as measures of uncertainty mapped to by any other candidate sampling location of the plurality of candidate sampling locations.

In some embodiments, receiving the one or more sample measurements corresponding to the one or more sampling locations comprises generating a request for the one or more sample measurements of soil at the one or more sampling locations. In some embodiments, the one or more soil characteristics comprise a measure of soil carbon content. In some embodiments, the measure of soil carbon content comprises a measure of soil organic carbon content. In some embodiments, the one or more covariates comprise at least one of: soil type, soil elevation, one or more climate measurements, and one or more optical measurements. In some embodiments, the one or more optical measurements comprise satellite imagery.

In some embodiments the soil mapping model comprises a machine learning model, the machine leaming model is configured to receive the one or more covariates as input and to generate predictions for the one or more soil characteristics as output, and the predictions are associated with a measure of confidence. Determining an uncertainty map for the soil mapping model comprises, for each of the plurality of locations, generating a prediction for the location and determining the measure of uncertainty for the location based on the measure of confidence for the prediction.

In some embodiments, the machine learning model comprises at least one of: a neural network and a random forest.

In some embodiments: the soil mapping model comprises an ensemble of soil sub-models; determining an uncertainty map for the soil mapping model comprises, for each of the plurality of locations, generating a prediction for the one or more soil characteristics at the location by each of the soil sub-models, thereby generating a plurality of predictions for the location, and determining the measure of uncertainty for the location based on the plurality of predictions. In some embodiments, a first soil sub-model of the ensemble comprises initial parameters trained over a first training dataset and a second soil sub-model of the ensemble comprises initial parameters trained over a second training dataset, the first and second datasets being disjoint.

In some embodiments, the first training dataset comprises images of the area of interest captured from an altitude of no more than 100 km and the second training dataset comprises images of the area of interest captured from an altitude of no less than 100 km. In some embodiments, the first training dataset comprises images captured by aircraft and the second training dataset comprises images captured by satellite. In some embodiments, the first training dataset comprises images having a first spatial density and the second training dataset comprises images having a second spatial density less than the first spatial density, such that an element of the second training dataset corresponds spatially to a plurality of elements of the first training dataset.

One aspect of the invention provides systems and methods for mapping soil characteristics. The system comprises one or more processors and a memory storing instructions which cause the one or more processors to perform operations comprising the method. The method is performed by a processor and comprises: mapping at least one location in an area of interest to at least one predicted value of at least one soil characteristics by a soil mapping model, the at least one predicted value based on a set of refined parameters for the soil mapping model trained according to any one of the training methods disclosed herein.

In addition to the exemplary aspects and embodiments described above, further aspects and embodiments will become apparent by reference to the drawings and by study of the following detailed descriptions.

### Brief Description of the Drawings

Exemplary embodiments are illustrated in referenced figures of the drawings. It is intended that the embodiments and figures disclosed herein are to be considered illustrative rather than restrictive.
Figure 1A shows schematically an exemplary system for training a soil mapping model in a first mode of operation for training a set of initial parameters for the soil mapping model.
Figure 1B shows schematically the exemplary system of Figure 1A in a second mode of operation for refining the parameters of the soil mapping model.
Figure 2 is a flowchart of an exemplary method for training a soil mapping model, such as the soil mapping model trained by the system of Figures 1A and 1B.
Figure 3 shows a first exemplary operating environment that includes at least one computing system for performing methods described herein, such as the method of Figure 2.

### Description

Throughout the following description specific details are set forth in order to provide a more thorough understanding to persons skilled in the art. However, well known elements may not have been shown or described in detail to avoid unnecessarily obscuring the disclosure. Accordingly, the description and drawings are to be regarded in an illustrative, rather than a restrictive, sense.

Aspects of the present disclosure provide systems and methods for training a soil mapping model, as well as systems and methods for generating soil maps with such trained soil map models which predict soil characteristics (e.g. carbon content) across an area of interest. In at least one embodiment, the training method involves refining the parameters of a soil map model by estimating the soil mapping model's uncertainty at various locations across an area of interest, selecting high-uncertainty areas, identifying sampling locations within those areas for further samples to be collected, and further training the parameters of the soil mapping model based on the newly-collected samples. Several techniques and suitable models for estimating uncertainty are disclosed.

By incorporating measures of uncertainty into the training process and collecting soil samples responsively to uncertainty, model performance can, in at least some circumstances, be improved with relatively fewer samples than might otherwise be obtained. This can be advantageous in many contexts; one context of particular interest is the collection of physical soil samples and analysis of such samples in a laboratory. Such samples can greatly reduce uncertainty but are often costly to obtain; the present disclosure's models and training techniques can allow for the targeting of such samples to high-uncertainty areas, with consequent effects on the quantity of training data required and/or on the performance of the soil mapping model.

The terms "uncertainty", "measure of uncertainty", "uncertainty map", and related terms, as used in this disclosure, include corresponding terms relating to certainty, confidence, or the like. For example, an embodiment may make use of a "measure of certainty" or generate a "confidence map" without departing from the present disclosure. Where uncertainty is compared between two things, such as where something is said to have "high uncertainty" or "greater uncertainty", this includes the meaning of having "low certainty" or "less certainty", respectively (and/or "low confidence" or "less confidence" or the like). Similarly, terms such as "low uncertainty" or "less uncertainty" include the meaning of having "high certainty" or "greater certainty", respectively (and/or "high confidence" or "greater confidence" or the like). To aid readability, and to avoid repetitively reminding the reader that (e.g.) measures of certainty, confidence, or the like may alternatively or additionally be used, the present disclosure and appended claims generally use "uncertainty" and related terms without loss of generality, except where the context requires otherwise.

### A System for Training a Soil Mapping Model

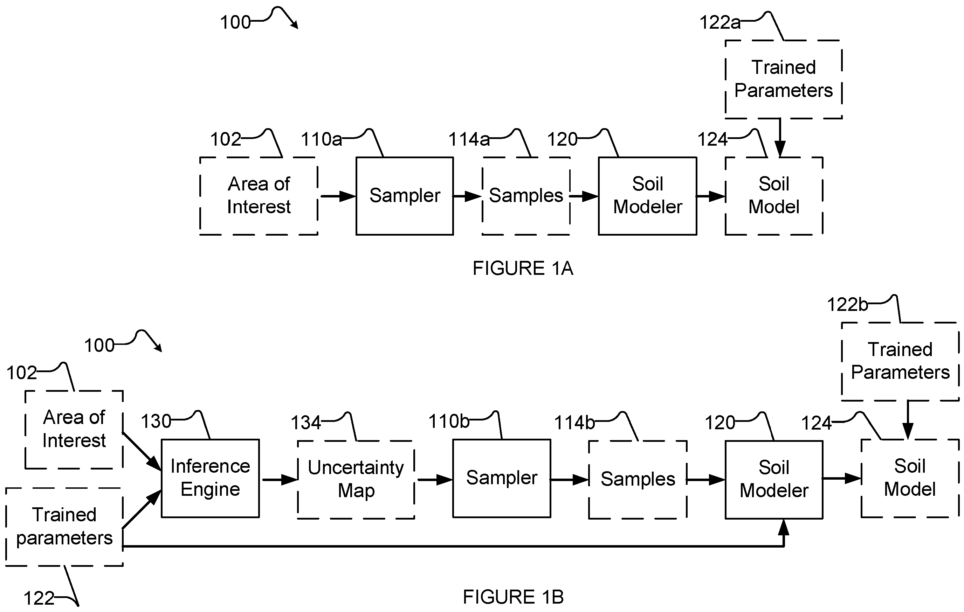

Figure 1A shows schematically an exemplary system 100 for training a soil mapping model 124 in a first mode of operation for training a set of initial trained parameters 122a (sometimes called "initial parameters" herein for convenience). Figure 1B shows system 100 in a second mode of operation for refining parameters of a soil mapping model such as soil mapping model 124. Figures 1A and 1B are collectively and individually referred to herein as "Figure 1". System 100 comprises a computing system as described in greater detail elsewhere herein. System 100 may interact with various inputs and outputs (shown in dashed lines), which are not necessarily part of system 100, although in some embodiments some or all of these inputs and outputs are part of system 100 (e.g. in an example embodiment, trained parameters 122a and/or 122b are part of system 100).

System 100 comprises a sampler 110a and a soil modeler 120, which in the first mode of operation shown in Figure 1A train a set of initial trained parameters 122a for soil mapping model 124. Sampler 110a receives a representation of an area of interest 102 and determines one or more sampling locations from which to obtain sample measurements 114a. Any suitable sampling approach may be used, such as random sampling, uniform sampling, stratified sampling, and/or conditioned Latin hypercube sampling. For example, sampler 110a may receive a map of an area together with ancillary variables describing features of the map (e.g. elevation, slope, etc.), and generate from that data one or more sampling locations based on a conditioned Latin hypercube sampling approach, e.g. as described by Minasny et al., A conditioned Latin hypercube method for sampling in the presence of ancillary information (2006), Comput. Geosci. 32, 9 (November, 2006), 1378-1388, doi:10.1016/j.cageo.2005.12.009. Sampler 110a may generate a request for one or more sample measurements 114a at the one or more sampling locations and receive sample measurements 114a from a user, another system, a sensor, etc. Sample measurements 114a may comprise any suitable measurements for use by soil modeler 120, such as spectral samples (e.g. obtained by satellite, aircraft, and/or terrestrial spectral sensors, such as imaging devices), physical measurements of physical soil samples (e.g. which may be chemically or otherwise analyzed to obtain ground-truth values for properties of interest such as carbon content, soil type, moisture, etc.).

Sample measurements 114a may be obtained by proximal and/or remote sensing. For example, sample measurements 114a may comprise physical measurements of physical soil samples, spectral data obtained by a proximal sensor (e.g. on a hand-held device, mounted on a terrestrial vehicle such as a rover, and/or otherwise proximally obtained), and/or other proximal sensor data of soil. Proximal sensor data may comprise data obtained of surface soil (e.g. by collecting surface soil and/or placing a sensor proximal to surface soil to collect measurements) and/or of sub-surface soil (e.g. by collecting sub-surface soil and/or by inserting a sensor into soil to collect measurements). Proximal and/or remote sensing may comprise, for example, the systems and methods described in greater detail in US provisional patent applications nos. 63/285,942 and 63/291,151.

Soil modeler 120 receives sample measurements 114a and generates a soil mapping model 124 having trained parameters 122a. Soil modeler 120 may generate any suitable type of soil mapping model 124 for relating one or more soil characteristics (e.g. carbon content, soil type, moisture) to one or more covariates over an area of interest. Soil characteristics may include, for example, features such as soil carbon content (e.g. soil organic carbon content), soil type, and/or moisture. Covariates may include, for example, slope, elevation, soil type, climate, image data (e.g. satellite imagery, drone imagery, terrestrial imagery, visual spectrum imagery, and/or infrared imagery), land use (e.g. agricultural, residential, forest, etc.), and/or farming practices (e.g. cover cropping, crop rotations, etc.). For example, soil modeler 120 may generate a linear estimation model which transforms covariate values to predicted values for the one or more soil characteristics based on weights trained by soil modeler 120 via a suitable training technique such as kriging and/or regression (e.g. geographically weighted regression). Alternatively, or in addition, soil modeler 120 may generate a non-linear model such as a neural network and/or a random forest having weights trained by soil modeler 120 via a suitable training technique such as backpropagation and/or induction/pruning. In at least some embodiments trained parameters 122a comprise such model weights. Examples of soil mapping models 124 and techniques for generating such models are provided by Mcbratney et al., On Digital Soil Mapping (2003), Geoderma, 117, 3-52, doi:10.1016/S0016-7061(03)00223-4.

System 100 refines trained parameters 122 (which may comprise initial parameters 122a and/or previously-refined parameters such as refined parameters 122b) of soil mapping model 124 in the second mode of operation, thereby training a set of refined trained parameters 122b. System 100 further comprises an inference engine 130 for generating an uncertainty map 134 based on soil mapping model 124 (e.g. based on the parameters of model 124, e.g. initial parameters 122a). Uncertainty map 134 maps locations in area of interest 102 to a measure of uncertainty.

Uncertainty may be estimated in any suitable way. In some embodiments, uncertainty is estimated based on a distribution in the output of the model. For example, in an embodiment where soil mapping model generates a predicted value for a soil characteristic at a given location, inference engine 130 may generate an uncertainty map 134 which maps that location to a measure of uncertainty in the predicted value for the soil characteristic. For instance, if soil mapping model 124 predicts a value c for soil carbon content at a location x, and the value c is associated with (e.g. a mean and/or mode of) a distribution of predicted values having variance v, then uncertainty map 134 may map location x to a value based on v - e.g. v itself and/or a combination of variances for a plurality of predicted values for soil characteristics (e.g. soil carbon content, moisture, soil type, etc.). As another example, uncertainty may be estimated by varying input parameters and measuring changes in model output (e.g. predictions); uncertainty may be estimated based on such variation, e.g. based on variance, standard deviation, average change, and/or any other suitable estimate. As yet a further example, uncertainty may be estimated via Monte Carlo methods, e.g. in the case where the model comprises a least-squares model.

As another example, soil mapping model 124 may comprise an ensemble of distinct soil mapping sub-models which produce different predicted values for a given soil characteristic; inference engine 130 may estimate uncertainty at a given location based on a variance (e.g. based on a standard deviation) of such values at the location. In some embodiments, the sub-models have distinct structure; e.g. one may comprise a linear model trained via kriging and another may comprise a neural network; such models may be trained over the same or different training datasets. In some embodiments, the sub-models are trained over different training datasets, such as where one is trained over images captured by a drone (e.g. at an altitude of no more than 100 km) and another is trained over images captured by satellite (e.g. at an altitude of no less than 100 km), and/or where one is trained over a first dataset having a certain spatial density and another is trained over a second dataset having a relatively denser spatial density (e.g. as may be the case when training one sub-model over high-resolution drone imagery with high spatial density, e.g. ~10 cm²/pixel, and training another sub-model over satellite imagery with relatively lower spatial density, e.g. 10 m²/pixel); such models may comprise the same or different model structures (e.g. all neural networks, all linear estimation models, other models, and/or combinations thereof). In some embodiments, an ensemble soil mapping model 124 comprises a random forest model with decision trees for sub-models; optionally, a sub-model may comprise a random forest.

Sampler 110b determines one or more sampling locations in the area of interest based on the uncertainty map. In some embodiments sampler 110a and sampler 110b are provided by a single sampler and/or sampler 110b may comprise sampler 110a. Sampler 110a and sampler 110b may be referred to collectively and individually as "sampler 110".

In some embodiments, area of interest 102 is divided into regions and sampler 110b selects one or more regions based on the uncertainty map - e.g. by selecting one or more regions having the greatest uncertainty relative to other regions. Area of interest 102 may be divided into regions by sampler 110b, by another element of system 100, and/or via a predetermined division. In some embodiments area of interest 102 is divided into rectilinear regions of predetermined number and/or size. In some embodiments area of interest 102 is divided into regions by clustering locations in area of interest 102 based on predicted soil characteristics, measures of uncertainty, and/or covariate measurements such that each region comprises a cluster. Sampler 110b may determine the uncertainty of a region based on uncertainty of one or more locations within the region; for instance, sampler 110b may determine the uncertainty of the region based on an aggregate measure, such as the mean, median or modal uncertainty of locations within the region. Sampler 110b may determine one or more sampling locations within the selected region(s) based on any suitable method. For example, sampler 110b of Figure 1B may optionally comprise sampler 110a of Figure 1A and/or may determine one or more sampling locations within the region substantially as described above with respect to sampler 110a, e.g. via random sampling, uniform sampling, stratified sampling, conditioned Latin hypercube sampling, and/or orthogonal sampling.

In some embodiments, sampler 110b determines one or more sampling locations by selecting sampling locations with high uncertainty. For example, sampler 110b may select one or more sampling locations from a set of candidate sampling locations, such as the set of all sampling locations in area of interest 102, sampling locations in certain regions (e.g. selected as described above), certain locations in each region (e.g. the location with greatest uncertainty in each region), and/or any other suitable set of candidate sampling locations. Sampler 110b may select from the set of candidate sampling locations one or more locations which are mapped to a measure of uncertainty by uncertainty map 134 which is at least as great as measures of uncertainty mapped to by any other (non-selected) candidate sampling location.

Sampler 110b may generate a request for one or more sample measurements 114b at the one or more sampling locations determined by sampler 110b. System 100 may receive sample measurements 114a from a user, another system, a sensor, etc. Sample measurements 114b may comprise any suitable measurements for use by soil modeler 120, as described elsewhere herein (e.g. with reference to sample measurements 114a). Sample measurements 114b characterize locations in area of interest 102 of high uncertainty (relative to other locations in area of interest 102) and are used by soil modeler 120 to refine parameters of soil mapping model 124 as described in greater detail below and elsewhere herein. In suitable circumstances, such refinement can efficiently reduce uncertainty in uncertainty map 134 and correspondingly improve accuracy and/or performance of soil mapping model 124.

Soil modeler 120 receives sample measurements 114b and refines parameters 122 to generate refined parameters 122b for soil mapping model 124. For example, soil modeler 120 may train soil mapping model 124 "from scratch" over a combination of sample measurements 114a and 114b to yield refined parameters 122b, e.g. by recalculating the weights of a linear estimation model over all sample measurements 114a and 114b collected thus far. As another example, soil modeler 120 may modify trained parameters 122 of soil mapping model 124 (which may comprise, e.g., initial parameters 122a and/or refined parameters 122b generated by a previous iteration of refinement) based on sample measurements 114b, e.g. by backpropagating over the weights of a neural network soil mapping model 124 based on sample measurements 114b and/or by otherwise retraining soil mapping model 124. For the purposes of this disclosure and the appended claims, all such generation of refined parameters 122b is considered to be "refining" parameters 122 whether or not parameters 122 are explicitly used by soil modeler 120, since samples 114b are themselves based on parameters 122 (via uncertainty map 134).

System 100 may iteratively refine parameters of soil model 124, e.g. by further refining refined parameters 122b. For instance, refined parameters 122b may take the position of parameters 122 in Figure 1B, such that inference engine 130 determines a further uncertainty map 134 based on refined parameters 122b, sampler 110b determines one or more further sampling locations in area of interest 102 based on the further uncertainty map 134, and soil modeler 120 receives one or more further sample measurements 114b corresponding to the one or more further sampling locations and generates further refined parameters 122b based on further sample measurements 114b. Such further refined parameters 122b may optionally be further refined as described herein.

In some embodiments, system 100 iteratively refines parameters 122 until a halting criterion is met. The halting criterion may comprise reaching a predetermined number of iterations, reaching a threshold measure of model uncertainty, and/or any other suitable halting criterion. For example, system 100 may determine a measure of model uncertainty based on at least one uncertainty maps 134, e.g. based on an average uncertainty across at least a portion of an uncertainty map. (For instance, measures of uncertainty at all locations in an uncertainty map 134 may be averaged to yield a measure of model uncertainty for soil mapping model 124 under trained parameters 122.) In some embodiments, system 100 halts iteration upon the measure of model uncertainty falling to below a threshold. In some embodiments, system 100 halts iteration upon a determination that the measure of model uncertainty is converging (e.g. remaining substantially unchanged between iterations, which may comprise changing by less than a threshold value, such as 1%, between iterations).

### A Method for Training a Soil Mapping Model

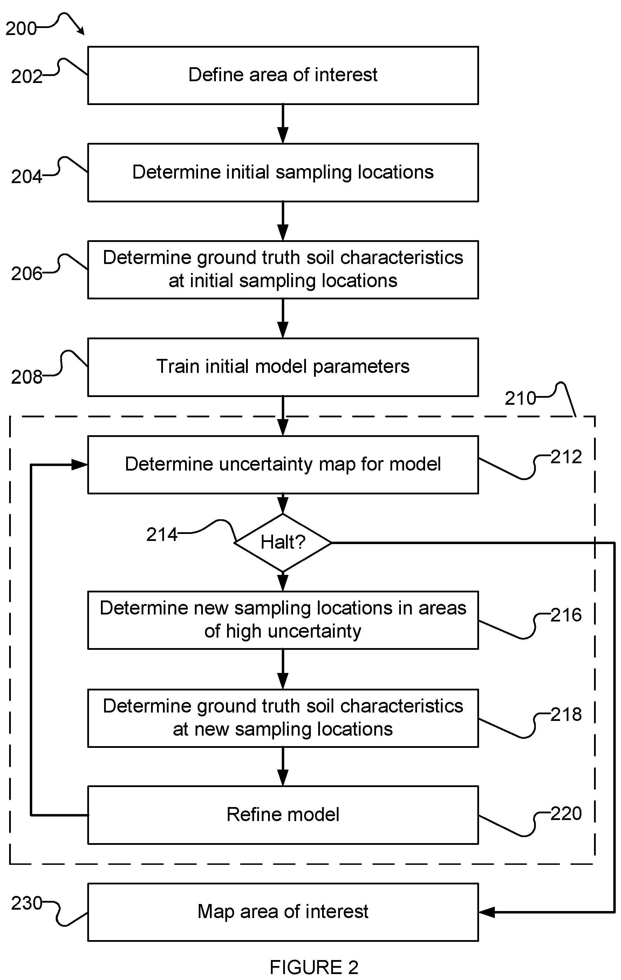

Figure 2 is a flowchart of an exemplary method 200 for training a soil mapping model, such as soil mapping model 124 trained by system 100 of Figures 1A and 1B. Method 200 is performed by a processor, such as a processor of computing system 300, described elsewhere herein.

Acts 202, 204, 206, and 208 are optional steps which generally relate to training a set of initial parameters for the soil mapping model. In some embodiments, the area of interest, initial sampling locations, initial training dataset, and/or initial parameters are predetermined or otherwise made available to the processor, in which case one or more of acts 202, 204, 206, and 208 may be omitted. In some such embodiments, the method commences at act 212 (and/or act 216, e.g. if the uncertainty map is also provided to the processor). Acts 202, 204, 206, and 208 are described in greater detail below.

At act 202, the processor defines an area of interest, such as by receiving a representation of the area of interest from a user, retrieving such a representation from a data store and/or from another system, generating the area of interest based on user input, and/or otherwise defining the area of interest. At act 204, the processor determines one or more initial sampling locations in the area of interest, e.g. by determining sampling locations by sampler 110a as described elsewhere herein. For instance, the one or more initial sampling locations may be determined according to conditioned Latin hypercube optimization across the area of interest. At act 206, the processor determines ground-truth soil characteristics at the one or more initial sampling locations, for example by obtaining and analyzing physical soil samples, imagery, and/or other information for the sampling locations, e.g. as described above with reference to sample measurements 114a, thereby providing an initial training dataset. At act 208, the processor trains a set of initial parameters for the soil mapping model based on the initial training dataset, e.g. by training via soil modeler 120 as described above. The soil mapping model is trained to relate one or more soil characteristics in the initial training dataset to one or more covariates over the area of interest.

At act 210, the processor refines parameters of the soil mapping model based on new samples collected at locations selected based on uncertainty in the soil mapping model so as to reduce such uncertainty. Act 210 may comprise various acts, e.g. as shown in Figure 2. For instance, act 210 may comprise act 212. At act 212, the processor determines an uncertainty map for the soil mapping model over at least a portion of the area of interest. The uncertainty map is determined based on the current parameters of the soil mapping model (e.g. initial parameters determined at act 208 and/or otherwise obtained). The uncertainty map maps locations in the area of interest to a measure of uncertainty in at least one of the one or more soil characteristics (e.g. soil carbon). For instance, the processor may generate the uncertainty map as described above with reference to inference engine 130 and the uncertainty map may map uncertainty as described above with reference to uncertainty map 134.

For the sake of example, let us consider a non-limiting implementation where the soil mapping model comprises a neural network with a set of trained parameters (e.g weights) which generates, for each location in the area of interest, a prediction for a soil characteristic, namely (in this example) soil carbon content. The prediction comprises a probability distribution over values describing the soil characteristic, e.g. via a softmax and/or other suitable transformation. The processor determines a measure of uncertainty for the location based on the probability distribution, e.g. based on a standard deviation, variance, width of a 95% confidence interval, and/or other suitable measure. The locations are, in this example, stored in memory in such a way as to be associated with their corresponding measures of uncertainty, e.g. as key-value pairs in a dictionary data structure, thereby providing an uncertainty map.

At act 214, the processor optionally decides whether to halt refinement. The processor may determine a halting criterion, e.g. as described above, and halt or continue accordingly. If the processor halts refinement, the method may proceed to act 230, described below. Otherwise, the method continues act 210, e.g. by proceeding to act 216.

At act 216, the processor determines one or more new sampling locations in the area of interest based on the uncertainty map. For instance, the processor may determine sampling locations as described above with reference to sampler 110b. At act 218, the processor receives one or more new sample measurements corresponding to the one or more new sampling locations, e.g. as described above with reference to sample measurements 114b.

At act 220, the processor refines the parameters of the soil mapping model (e.g. initial parameters determined at 208, previously refined via earlier iterations of act 220, and/or otherwise obtained) based on the one or more new sample measurements to generate a set of refined parameters for the soil mapping model. For instance, the processor may refine the parameters of the soil mapping model as described above with reference to soil modeler 120, soil model 124, and refined parameters 122b.

In some embodiments (including the depicted embodiment of Figure 2), method 200 returns from act 220 to act 212. For instance, method 200 may return from act 220 to act 212, determine a new uncertainty map, and (at act 214) determine whether to halt or continue iterating act 210 based on the new uncertainty map. In some embodiments, method 200 proceeds from act 220 to act 230. For instance, the processor may halt iteration of act 210 after a predetermined number of iterations and may check whether the predetermined number of iterations has elapsed following act 220.

At act 230, the processor maps at least one location in the area of interest to at least one predicted value of at least one of the one or more soil characteristics based on the refined parameters. Act 230 is optional while training the soil mapping model, and may be performed separately from the training acts of method 200 (e.g. all preceding acts). Aspects of act 230 are described in greater detail below.

### Generating Soil Maps with Trained Soil Mapping Models

The soil mapping model may be used to map the area of interest based on the refined parameters. For example, a processor of a computing system may receive one or more covariate values and transform the covariate values with the soil mapping model, based on refined parameters of the soil mapping model (e.g. as generated at act 220), into a predicted soil characteristic value. Covariates may include any suitable covariates such as those described elsewhere herein, e.g. elevation and/or imagery (e.g. infrared reflectance imagery). Soil characteristics may include any suitable characteristics such as those described elsewhere herein, e.g. soil carbon content. The processor may generate predicted values for soil characteristics at one, some, and/or all locations in the area of interest and/or portions thereof in any manner suitable for the soil mapping model. For instance, the predicted values may be generated via inference with a neural network soil mapping model, via linear transformation with a linear estimation model, etc. The processor may, but does not necessarily, generate an uncertainty map as part of this mapping procedure. The processor does not necessarily perform any of the training acts of method 200 (i.e. all acts other than act 230), although these may optionally also be performed.

### Example System Implementation

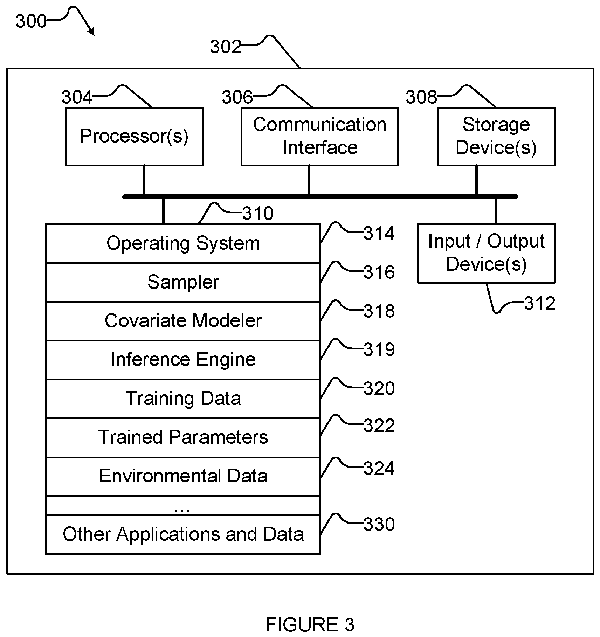

Figure 3 illustrates a first exemplary operating environment 300 that includes at least one computing system 302 for performing methods described herein. System 302 may be any suitable type of electronic device, such as, without limitation, a mobile device, a personal digital assistant, a mobile computing device, a smart phone, a cellular telephone, a handheld computer, a server, a server array or server farm, a web server, a network server, a blade server, an Internet server, a work station, a mini-computer, a mainframe computer, a supercomputer, a network appliance, a web appliance, a distributed computing system, multiprocessor systems, or combination thereof. System 302 may be configured in a network environment, a distributed environment, a multi-processor environment, and/or a stand-alone computing device having access to remote or local storage devices.

A computing system 302 may include one or more processors 304, a communication interface 306, one or more storage devices 308, one or more input and output devices 312, and a memory 310. A processor 304 may be any commercially available or customized processor and may include dual microprocessors and multi-processor architectures. The communication interface 306 facilitates wired or wireless communications between the computing system 302 and other devices. A storage device 308 may be a computer-readable medium that does not contain propagating signals, such as modulated data signals transmitted through a carrier wave. Examples of a storage device 308 include without limitation RAM, ROM, EEPROM, flash memory or other memory technology, CD-ROM, digital versatile disks (DVD), or other optical storage, magnetic cassettes, magnetic tape, magnetic disk storage. In at least some embodiments such embodiments of storage device 308 do not contain propagating signals, such as modulated data signals transmitted through a carrier wave. There may be multiple storage devices 308 in the computing system 302. The input/output devices 312 may include a keyboard, mouse, pen, voice input device, touch input device, display, speakers, printers, etc., and any combination thereof.

The memory 310 may be any non-transitory computer-readable storage media that may store executable procedures, applications, and data. The computer-readable storage media does not pertain to propagated signals, such as modulated data signals transmitted through a carrier wave. It may be any type of non-transitory memory device (e.g., random access memory, read-only memory, etc.), magnetic storage, volatile storage, non-volatile storage, optical storage, DVD, CD, floppy disk drive, etc. that does not pertain to propagated signals, such as modulated data signals transmitted through a carrier wave. The memory 310 may also include one or more external storage devices or remotely located storage devices that do not pertain to propagated signals, such as modulated data signals transmitted through a carrier wave.

The memory 310 may contain instructions, components, and data. A component is a software program that performs a specific function and is otherwise known as a module, program, engine, and/or application. The memory 310 may include an operating system 314, a sampler 316, a covariate modeler 318, an inference engine 319, training data 320 (e.g. one or more plant images and/or ground-truth structural representations), trained parameters 322 (e.g. comprising parameters 122), one or more input images 324 (e.g. plant images), and other applications and data 330. Depending on the embodiment, some such elements may be wholly or partially omitted. For example, an embodiment intended for inference (e.g. via method 200 and/or system 302) and which has trained parameters 322 might omit training data 320, decoder 318, discriminator 319, and/or some or all trained parameters 322 other than those required by sampler 316. As another example, memory 310 may include no images 324 prior to starting inference (e.g. via system 302 and/or method 200) and may receive images (one-by-one and/or in batches) via an input device 312 and/or from a storage device 308.

While a number of exemplary aspects and embodiments have been discussed above, those of skill in the art will recognize certain modifications, permutations, additions and subcombinations thereof. The scope of the inventions is however defined by the following appended claims.

## Claims

1. A method for training a soil mapping model, the method performed by a processor and comprising:
training (208) a set of initial parameters for a soil mapping model relating one or more soil characteristics to one or more covariates over an area of interest (102) based on an initial training dataset;
determining (212) a first uncertainty map (134) for the soil mapping model over at least a portion of the area of interest (102) based on the initial parameters, the first uncertainty map (134) mapping each of a plurality of locations in the area of interest (102) to a measure of uncertainty in at least one of the one or more soil characteristics;
determining (216) one or more primary sampling locations in the area of interest (102) based on the first uncertainty map (134) comprising selecting a first one of a plurality of candidate sampling locations, the first candidate sampling location being mapped to a measure of uncertainty by the first uncertainty map at least as great as measures of uncertainty mapped to by any other candidate sampling location of the plurality of candidate sampling locations;
receiving (218) one or more sample measurements corresponding to the first one of the plurality of candidate sampling locations;
refining (220) the set of initial parameters of the soil mapping model based on the one or more sample measurements to generate a first set of refined parameters for the soil mapping model;
determining (212) a second uncertainty map (134), the second uncertainty map (134) mapping each of a plurality of locations in the area of interest (102) to a second measure of uncertainty in at least one of the one or more soil characteristics;
determining (216) one or more secondary sampling locations in the area of interest (102) based on the second uncertainty map (134) comprising selecting a second one of a plurality of candidate sampling locations, the second candidate sampling location being mapped to a measure of uncertainty by the second uncertainty map (134) at least as great as measures of uncertainty mapped to by any other candidate sampling location of the plurality of candidate sampling locations;
receiving (218) one or more sample measurements corresponding to the second one of the plurality of secondary sampling locations; and
refining (220) the first set of refined parameters of the soil mapping model based on the one or more measurements to generate a second set of refined parameters for the soil mapping model.

2. The method according to claim 1 comprising mapping (230) at least one location in the area of interest (102) to at least one predicted value of at least one of the one or more soil characteristics, the at least one predicted value based on at least one of: the first set of refined parameters and a further set of refined parameters based on the first set of refined parameters for the soil mapping model.

3. The method according to claim 1 comprising iteratively generating one or more further sets of refined parameters, each set of refined parameters generated by:
determining (212) a further uncertainty map (134) for the soil mapping model based on at least one of the first set of refined parameters and the one or more further sets of refined parameters;
determining (216) one or more further sampling locations in the area of interest (102) based on the further uncertainty map (134);
receiving (218) one or more further sample measurements corresponding to the one or more further sampling locations; and
refining (220) at least one of the first set of refined parameters and at least one further sets of refined parameters based on the one or more sample measurements to generate a further set of refined parameters for the soil mapping model.

4. The method according to claim 3 comprising mapping (230) at least one location in the area of interest (102) to at least one predicted value of at least one of the one or more soil characteristics, the at least one predicted value based on at least one of the further sets of refined parameters for the soil mapping model.

5. The method according to claim 3 wherein iteratively generating the one or more further sets of refined parameters comprises determining a measure of model uncertainty based on at least one of the uncertainty maps (134); and halting generation of further refined parameters based on the measure of model uncertainty; optionally wherein the measure of model uncertainty comprises an average of measures of uncertainty for the plurality of locations in the area of interest (102); and optionally wherein halting generation of further refined parameters based on the measure of model uncertainty comprises determining that the measure of model uncertainty is less than a threshold uncertainty value.

6. The method according to claim 1 wherein:
the plurality of locations mapped by the uncertainty map (134) comprises a plurality of regions in the area of interest (102); and
determining (216) the one or more sampling locations comprises at least one of: random sampling, uniform sampling, stratified sampling, and conditioned Latin hypercube sampling, and orthogonal sampling in the at least one region; and
optionally determining (216) the one or more sampling locations comprises determining, for each one of the plurality of regions, a measure of aggregate uncertainty for the region; and selecting the at least one region based on the measures of aggregate uncertainty.

7. The method according to claim 1 comprising measuring soil at the one or more sampling locations to generate the one or more sample measurements; optionally wherein measuring soil at the one or more sampling locations comprises generating the one or more sample measurements by proximal sensing; further optionally wherein generating the one or more sample measurements by proximal sensing comprises inserting a proximal sensor into soil at the one or more sampling locations.

8. The method according to claim 1 wherein the one or more soil characteristics comprise a measure of soil carbon content; optionally wherein the measure of soil carbon content comprises a measure of soil organic carbon content.

9. The method according to claim 1 wherein the one or more covariates comprise at least one of: soil type, soil elevation, one or more climate measurements, one or more land uses, one or more farming practices, and one or more optical measurements; optionally wherein the one or more optical measurements comprise satellite imagery.

10. The method according to claim 1 wherein:
the soil mapping model comprises a machine learning model, the machine learning model configured to receive the one or more covariates as input and to generate predictions for the one or more soil characteristics as output, the predictions associated with a measure of confidence; and
determining (216) an uncertainty map (134) for the soil mapping model comprises, for each of the plurality of locations, generating a prediction for the location and determining the measure of uncertainty for the location based on the measure of confidence for the prediction; and
optionally wherein the machine learning model comprises at least one of: a neural network and a random forest.

11. The method according to claim 1 wherein:
the soil mapping model comprises an ensemble of soil sub-models;
determining (216) an uncertainty map (134) for the soil mapping model comprises, for each of the plurality of locations, generating a prediction for the one or more soil characteristics at the location by each of the soil sub-models, thereby generating a plurality of predictions for the location, and determining the measure of uncertainty for the location based on the plurality of predictions;
optionally wherein a first soil sub-model of the ensemble comprises initial parameters trained over a first training dataset and a second soil sub-model of the ensemble comprises initial parameters trained over a second training dataset, the first and second datasets being disjoint.

12. The method according to claim 11 wherein the first training dataset comprises images having a first spatial density and the second training dataset comprises images having a second spatial density less than the first spatial density, such that an element of the second training dataset corresponds spatially to a plurality of elements of the first training dataset.

13. A method for mapping soil characteristics, the method performed by a processor and comprising:
mapping at least one location in an area of interest (102) to at least one predicted value of at least one soil characteristics by a soil mapping model, the at least one predicted value based on a set of at least first and second refined parameters for the soil mapping model trained according to any one of claims 1 to 12.

14. A computer system comprising:
one or more processors; and
a memory storing instructions which cause the one or more processors to perform operations comprising the method of any one of claims 1 to 13.

## Patentansprüche

1. Verfahren zum Trainieren eines Bodenkartierungsmodells, wobei das Verfahren durch einen Prozessor durchgeführt wird, und Folgendes umfasst:
Trainieren (208) eines Satz von Anfangsparametern für ein Bodenkartierungsmodell, das sich auf eine oder mehrere Bodeneigenschaften mit einer oder mehreren Kovariablen über ein Interessengebiet (102) basierend auf einem anfänglichen Trainingsdatensatzes bezieht;
Bestimmen (212) einer ersten Unsicherheitskarte (134) für das Bodenkartierungsmodell über mindestens einen Abschnitt des Interessengebiets (102) basierend auf dem Anfangsparameter, wobei die erste Unsicherheitskarte (134) jeden von einer Vielzahl von Stellen in dem Interessengebiet (102) auf ein Unsicherheitsmaß in mindestens einer der einen oder mehreren Bodeneigenschaften kartiert;
Bestimmen (216) einer oder mehrerer primärer Probenahmestellen in dem Interessengebiet (102) basierend auf der ersten Unsicherheitskarte (134), umfassend Auswählen einer ersten von einer Vielzahl von Kandidaten-Probenahmestellen, wobei die erste Kandidaten-Probenahmestelle auf ein Unsicherheitsmaß durch die erste Unsicherheitskarte kartiert wird, das mindestens so groß wie die Unsicherheitsmaße ist, auf die durch eine beliebige andere Kandidaten-Probenahmestelle der Vielzahl von Kandidaten-Probenahmestellen kartiert wird;
Empfangen (218) einer oder mehrerer Probenmessungen entsprechend der ersten von der Vielzahl von Kandidaten-Probenahmestellen;
Verfeinern (220) des Satzes von Anfangsparametern des Bodenkartierungsmodells basierend auf der einen oder den mehreren Probenmessungen, um einen ersten Satz von verfeinerten Parametern für das Bodenkartierungsmodell zu generieren;
Bestimmen (212) einer zweiten Unsicherheitskarte (134), wobei die zweite Unsicherheitskarte (134) jeden von einer Vielzahl von Stellen in dem Interessengebiet (102) auf ein zweites Unsicherheitsmaß in mindestens einer der einen oder mehreren Bodeneigenschaften kartiert;
Bestimmen (216) einer oder mehrerer sekundärer Probenahmestellen in dem Interessengebiet (102) basierend auf der zweiten Unsicherheitskarte (134), umfassend Auswählen einer zweiten von einer Vielzahl von Kandidaten-Probenahmestellen, wobei die zweite Kandidaten-Probenahmestelle auf ein Unsicherheitsmaß durch die zweite Unsicherheitskarte (134) kartiert wird, das mindestens so groß wie die Unsicherheitsmaße ist, auf die durch eine beliebige andere Kandidaten-Probenahmestelle der Vielzahl von Kandidaten-Probenahmestellen kartiert wird;
Empfangen (218) einer oder mehrerer Probenmessungen entsprechend der zweiten von der Vielzahl von sekundären Probenahmestellen; und
Verfeinern (220) des ersten Satzes von verfeinerten Parametern des Bodenkartierungsmodells basierend auf der einen oder den mehreren Messungen, um einen zweiten Satz von verfeinerten Parametern für das Bodenkartierungsmodell zu generieren.

2. Verfahren nach Anspruch 1, umfassend Kartieren (230) mindestens einer Stelle in dem Interessengebiet (102) auf mindestens einem vorhergesagten Wert von mindestens einer der einen oder mehreren Bodeneigenschaften, wobei der mindestens eine vorhergesagte Wert auf mindestens einem der folgenden Elemente basiert: dem ersten Satz von verfeinerten Parametern und einem weiteren Satz von verfeinerten Parametern basierend auf dem ersten Satz von verfeinerten Parametern für das Bodenkartierungsmodell.

3. Verfahren nach Anspruch 1, umfassend iteratives Generieren eines oder mehrerer weiterer Sätze von verfeinerten Parametern, wobei jeder Satz von verfeinerten Parametern durch Folgendes generiert wird:
Bestimmen (212) einer weiteren Unsicherheitskarte (134) für das Bodenkartierungsmodell basierend auf dem mindestens einen des ersten Satzes von verfeinerten Parametern und des einen oder der mehreren weiteren Sätze von verfeinerten Parametern;
Bestimmen (216) einer oder mehrerer weiterer Probenahmestellen in dem Interessengebiet (102) basierend auf der weiteren Unsicherheitskarte (134);
Empfangen (218) einer oder mehrerer weiterer Probenmessungen entsprechend der einen oder den mehreren weiteren Probenahmestellen; und
Verfeinern (220) mindestens des ersten Satzes von verfeinerten Parametern und mindestens eines weiteren Satzes von verfeinerten Parametern basierend auf der einen oder den mehreren Probemessungen, um einen weiteren Satz von verfeinerten Parametern für das Bodenkartierungsmodell zu generieren.

4. Verfahren nach Anspruch 3, umfassend Kartieren (230) mindestens einer Stelle in dem Interessengebiet (102) auf mindestens einem vorhergesagten Wert von mindestens einer der einen oder mehreren Bodeneigenschaften, wobei der mindestens eine vorhergesagte Wert auf mindestens einem der weiteren Sätze von verfeinerten Parametern für das Bodenkartierungsmodell basiert.

5. Verfahren nach Anspruch 3, wobei das iterative Generieren des einen oder der mehreren weiteren Sätze von verfeinerten Parametern das Bestimmen eines Modellunsicherheitsmaßes basierend auf mindestens einer der Unsicherheitskarten (134) umfasst; und Anhalten der Generierung weiterer verfeinerter Parameter basierend auf dem Modellunsicherheitsmaß umfasst; wobei das Modellunsicherheitsmaß optional einen Mittelwert der Unsicherheitsmaße für die Vielzahl von Stellen in dem Interessengebiet (102) umfasst; und wobei das Anhalten der Generierung weiterer verfeinerter Parameter basierend auf dem Modellunsicherheitsmaß optional das Bestimmen umfasst, dass das Modellunsicherheitsmaß kleiner als ein Schwellenwertunsicherheitswert ist.

6. Verfahren nach Anspruch 1, wobei:
die Vielzahl von Stellen, die durch die Unsicherheitskarte (134) kartiert sind, eine Vielzahl von Regionen in dem Interessengebiet (102) umfasst; und
das Bestimmen (216) der einen oder der mehreren Probenahmestellen mindestens eines des Folgenden umfasst:
Entnahme von Zufallsstichproben, gleichmäßigen Stichproben, geschichteten Stichproben und konditionierten Latin-Hypercube-Stichproben sowie orthogonalen Stichproben in der mindestens einen Region; und
optionales Bestimmen (216) der einen oder mehreren Probenahmestellen das Bestimmen eines Gesamtunsicherheitsmaßes für die Region für jede der Vielzahl von Regionen umfasst; und Auswählen der mindestens einen Region basierend auf den Gesamtunsicherheitsmaßen.

7. Verfahren nach Anspruch 1, umfassend Messen des Bodens an der einen oder den mehreren Probenahmestellen, um die eine oder mehreren Probenmessungen zu generieren; wobei das Messen des Bodens an der einen oder den mehreren Probenahmestellen optional das Generieren der einen oder mehreren Probenmessungen durch Nahfeldmessung umfasst; wobei das Generieren der einen oder mehreren Probenmessungen durch Nahfeldmessung ferner optional das Einführen eines Nahfeldsensors in den Boden an der einen oder den mehreren Probenahmestellen umfasst.

8. Verfahren nach Anspruch 1, wobei die eine oder mehreren Bodeneigenschaften ein Maß für den Kohlenstoffgehalt im Boden umfassen; wobei das Maß für den Kohlenstoffgehalt im Boden optional ein Maß für den Gehalt an organischem Kohlenstoff im Boden umfasst.

9. Verfahren nach Anspruch 1, wobei die eine oder mehreren Kovariablen mindestens eine der folgenden Eigenschaften umfassen: Bodentyp, Bodenhöhe, eine oder mehrere Klimamessungen, eine oder mehrere Landnutzungen, eine oder mehrere landwirtschaftliche Praktiken und eine oder mehrere optische Messungen; wobei die eine oder mehreren optischen Messungen optional Satellitenbilder umfassen.

10. Verfahren nach Anspruch 1, wobei:
das Bodenkartierungsmodell ein Maschinenlernmodell umfasst, das Maschinenlernmodell dazu konfiguriert ist, die eine oder mehreren Kovariaten als Eingabe zu empfangen und Vorhersagen für die eine oder mehreren Bodeneigenschaften als Ausgabe zu generieren, wobei die Vorhersagen mit einem Vertrauensmaß assoziiert sind; und
das Bestimmen (216) einer Unsicherheitskarte (134) für das Bodenkartierungsmodell für jede der Vielzahl von Stellen das Generieren einer Vorhersage für die Stelle und das Bestimmen des Unsicherheitsmaßes für die Stelle basierend auf dem Vertrauensmaß für die Vorhersage umfasst; und
wobei das Maschinenlernmodell optional mindestens eines der folgenden Elemente umfasst: ein neuronales Netzwerk und einen Random Forest.

11. Verfahren nach Anspruch 1, wobei:
das Bodenkartierungsmodell ein Ensemble von Bodenteilmodellen umfasst;
das Bestimmen (216) einer Unsicherheitskarte (134) für das Bodenkartierungsmodell für jede der Vielzahl von Stellen das Generieren einer Vorhersage für die eine oder mehreren Bodeneigenschaften an der Stelle durch jedes der Bodenteilmodelle, dadurch das Generieren einer Vielzahl von Vorhersagen für die Stelle und das Bestimmen des Unsicherheitsmaßes für die Stelle basierend auf der Vielzahl von Vorhersagen umfasst;
wobei ein erstes Bodenteilmodell des Ensembles optional Anfangsparameter umfasst, die über einen ersten Trainingsdatensatz trainiert werden, und ein zweites Bodenteilmodell des Ensembles optional Anfangsparameter umfasst, die über einen zweiten Trainingsdatensatz trainiert werden, wobei der erste und der zweite Datensatz nicht zusammenhängen.

12. Verfahren nach Anspruch 11, wobei der erste Trainingsdatensatz Bilder umfasst, die eine erste räumliche Dichte aufweisen, und der zweite Trainingsdatensatz Bilder umfasst, die eine zweite räumliche Dichte aufweisen, die derart kleiner als die erste räumliche Dichte ist, dass ein Element des zweiten Trainingsdatensatzes räumlich einer Vielzahl von Elementen des ersten Trainingsdatensatzes entspricht.

13. Verfahren zum Kartieren von Bodeneigenschaften, wobei das Verfahren durch einen Prozessor durchgeführt wird, und Folgendes umfasst:
Kartieren mindestens einer Stelle in einem Interessengebiet (102) auf mindestens einem vorhergesagten Wert von mindestens einer Bodeneigenschaft durch ein Bodenkartierungsmodell, wobei der mindestens eine vorhergesagte Wert auf einem Satz von mindestens ersten und zweiten verfeinerten Paramatern für das Bodenkartierungsmodell basiert, das nach einem der Ansprüche 1 bis 12 trainiert wird.

14. Computersystem, umfassend:
einen oder mehrere Prozessoren; und
einen Speicher, der Anweisungen speichert, welche bewirken, dass der eine oder die mehreren Prozessoren Operationen, umfassend das Verfahren nach einem der Ansprüche 1 bis 13, durchführen.

## Revendications

1. Procédé d'entraînement d'un modèle de cartographie du sol, le procédé étant mis en œuvre par un processeur et comprenant :
l'entraînement (208) d'un ensemble de paramètres initiaux pour un modèle de cartographie du sol reliant une ou plusieurs caractéristiques du sol à une ou plusieurs covariables sur une zone d'intérêt (102) sur la base d'un ensemble de données d'entraînement initial ;
la détermination (212) d'une première carte d'incertitude (134) pour le modèle de cartographie du sol sur au moins une partie de la zone d'intérêt (102) sur la base des paramètres initiaux, la première carte d'incertitude (134) cartographiant chacun d'une pluralité d'emplacements dans la zone d'intérêt (102) à une mesure d'incertitude dans au moins l'une des une ou plusieurs caractéristiques du sol ;
la détermination (216) d'un ou de plusieurs emplacements d'échantillonnage primaires dans la zone d'intérêt (102) sur la base de la première carte d'incertitude (134) comprenant la sélection d'un premier parmi une pluralité d'emplacements d'échantillonnage candidats, le premier emplacement d'échantillonnage candidat étant associé à une mesure d'incertitude par la première carte d'incertitude au moins aussi grande que des mesures d'incertitude associées à tout autre emplacement d'échantillonnage candidat parmi la pluralité d'emplacements d'échantillonnage candidats ;
la réception (218) d'une ou de plusieurs mesures d'échantillonnage correspondant au premier parmi la pluralité d'emplacements d'échantillonnage candidats ;
le raffinement (220) de l'ensemble de paramètres initiaux du modèle de cartographie du sol sur la base des une ou plusieurs mesures d'échantillonnage pour générer un premier ensemble de paramètres raffinés pour le modèle de cartographie du sol ;
la détermination (212) d'une seconde carte d'incertitude (134), la seconde carte d'incertitude (134) cartographiant chacun d'une pluralité d'emplacements dans la zone d'intérêt (102) à une seconde mesure d'incertitude dans au moins l'une des une ou plusieurs caractéristiques du sol ;
la détermination (216) d'un ou de plusieurs emplacements d'échantillonnage secondaires dans la zone d'intérêt (102) sur la base de la seconde carte d'incertitude (134) comprenant la sélection d'un second parmi une pluralité d'emplacements d'échantillonnage candidats, le second emplacement d'échantillonnage candidat étant associé à une mesure d'incertitude par la seconde carte d'incertitude (134) au moins aussi grande que des mesures d'incertitude associées à tout autre emplacement d'échantillonnage candidat parmi la pluralité d'emplacements d'échantillonnage candidats ;
la réception (218) d'une ou de plusieurs mesures d'échantillonnage correspondant au second parmi la pluralité d'emplacements d'échantillonnage secondaires ; et
le raffinement (220) du premier ensemble de paramètres raffinés du modèle de cartographie du sol sur la base des une ou plusieurs mesures pour générer un second ensemble de paramètres raffinés pour le modèle de cartographie du sol.

2. Procédé selon la revendication 1 comprenant la cartographie (230) d'au moins un emplacement dans la zone d'intérêt (102) à au moins une valeur prédite d'au moins une des une ou plusieurs caractéristiques du sol, l'au moins une valeur prédites basée sur au moins un des éléments suivants : le premier ensemble de paramètres raffinés et un autre ensemble de paramètres raffinés sur la base du premier ensemble de paramètres raffinés pour le modèle de cartographie du sol.

3. Procédé selon la revendication 1 comprenant la génération itérative d'un ou de plusieurs ensembles supplémentaires de paramètres raffinés, chaque ensemble de paramètres raffinés étant généré par :
la détermination (212) d'une carte d'incertitude supplémentaire (134) pour le modèle de cartographie du sol sur la base d'au moins l'un du premier ensemble de paramètres raffinés et des un ou plusieurs autres ensembles de paramètres raffinés ;
la détermination (216) d'un ou de plusieurs emplacements d'échantillonnage supplémentaires dans la zone d'intérêt (102) sur la base de la carte d'incertitude supplémentaire (134) ;
la réception (218) d'une ou de plusieurs mesures d'échantillonnage supplémentaires correspondant aux un ou plusieurs emplacements d'échantillonnage supplémentaires ; et
le raffinement (220) d'au moins l'un du premier ensemble de paramètres raffinés et d'au moins un autre ensemble de paramètres raffinés sur la base des une ou plusieurs mesures d'échantillonnage pour générer un ensemble de paramètres raffinés supplémentaire pour le modèle de cartographie du sol.

4. Procédé selon la revendication 3 comprenant la cartographie (230) d'au moins un emplacement dans la zone d'intérêt (102) à au moins une valeur prédite d'au moins une des une ou plusieurs caractéristiques du sol, l'au moins une valeur prédites basée sur au moins l'un des ensembles de paramètres raffinés supplémentaires pour le modèle de cartographie du sol.

5. Procédé selon la revendication 3 dans lequel la génération itérative des un ou plusieurs ensembles supplémentaires de paramètres raffinés comprend la détermination d'une mesure d'incertitude de modèle sur la base d'au moins une des cartes d'incertitude (134) ; et l'arrêt de la génération de paramètres raffinés supplémentaires sur la base de la mesure d'incertitude de modèle ; éventuellement dans lequel la mesure d'incertitude de modèle comprend une moyenne des mesures d'incertitude pour la pluralité d'emplacements dans la zone d'intérêt (102) ; et éventuellement dans lequel l'arrêt de la génération de paramètres raffinés supplémentaires sur la base de la mesure d'incertitude de modèle comprend la détermination selon laquelle la mesure d'incertitude de modèle est inférieure à une valeur d'incertitude seuil.

6. Procédé selon la revendication 1 dans lequel :
la pluralité d'emplacements cartographiés par la carte d'incertitude (134) comprend une pluralité de régions dans la zone d'intérêt (102) ; et
la détermination (216) des un ou plusieurs emplacements d'échantillonnage comprend au moins l'une des méthodes suivantes : l'échantillonnage aléatoire, l'échantillonnage uniforme, l'échantillonnage stratifié, l'échantillonnage hypercube latin conditionné et l'échantillonnage orthogonal dans l'au moins une région ; et
la détermination (216) éventuelle des un ou plusieurs emplacements d'échantillonnage comprend la détermination, pour chacune des régions, d'une mesure d'incertitude agrégée pour la région ; et la sélection de l'au moins une région sur la base des mesures d'incertitude agrégée.

7. Procédé selon la revendication 1 comprenant la mesure du sol aux un ou plusieurs emplacements d'échantillonnage pour générer les une ou plusieurs mesures d'échantillonnage ; éventuellement dans lequel la mesure du sol aux un ou plusieurs emplacements d'échantillonnage comprend la génération des une ou plusieurs mesures d'échantillonnage par détection de proximité ; plus éventuellement dans lequel la génération des une ou plusieurs mesures d'échantillon par détection de proximité comprend l'insertion d'un capteur de proximité dans le sol aux un ou plusieurs emplacements d'échantillonnage.

8. Procédé selon la revendication 1, dans lequel les une ou plusieurs caractéristiques du sol comprennent une mesure de la teneur en carbone du sol ; éventuellement dans lequel la mesure de la teneur en carbone du sol comprend une mesure de la teneur en carbone organique du sol.

9. Procédé selon la revendication 1, dans lequel les un ou plusieurs covariables comprennent au moins l'un des éléments suivants : le type de sol, l'altitude du sol, une ou plusieurs mesures climatiques, une ou plusieurs utilisations des terres, une ou plusieurs pratiques agricoles et une ou plusieurs mesures optiques ; éventuellement dans lequel les une ou plusieurs mesures optiques comprennent des images satellites.

10. Procédé selon la revendication 1 dans lequel :
le modèle de cartographie du sol comprend un modèle d'apprentissage automatique, le modèle d'apprentissage automatique étant configuré pour recevoir les une ou plusieurs covariables en entrée et pour générer des prédictions pour les une ou plusieurs caractéristiques du sol en sortie, les prédictions étant associées à une mesure de confiance ; et
la détermination (216) d'une carte d'incertitude (134) pour le modèle de cartographie du sol comprend, pour chacun de la pluralité d'emplacements, la génération d'une prédiction pour l'emplacement et la détermination de la mesure d'incertitude pour l'emplacement sur la base de la mesure de confiance de la prédiction ; et
éventuellement dans lequel le modèle d'apprentissage automatique comprend au moins un des éléments suivants : un réseau neuronal et une forêt aléatoire.

11. Procédé selon la revendication 1 dans lequel :
le modèle de cartographie du sol comprend un ensemble de sous-modèles de sol ;
la détermination (216) d'une carte d'incertitude (134) pour le modèle de cartographie du sol comprend, pour chacun de la pluralité d'emplacements, la génération d'une prédiction pour les une ou plusieurs caractéristiques de sol à l'emplacement par chacun des sous-modèles de sol, et ainsi la génération d'une pluralité de prédictions pour l'emplacement, et la détermination de la mesure d'incertitude pour l'emplacement sur la base de la pluralité de prédictions ;
éventuellement dans lequel un premier sous-modèle de sol de l'ensemble comprend des paramètres initiaux entraînés sur un premier ensemble de données d'entraînement et un second sous-modèle de sol de l'ensemble comprend des paramètres initiaux entraînés sur un second ensemble de données d'entraînement, les premier et second ensembles de données étant disjoints.

12. Procédé selon la revendication 11 dans lequel le premier ensemble de données d'entraînement comprend des images présentant une première densité spatiale et le second ensemble de données d'entraînement comprend des images présentant une seconde densité spatiale inférieure à la première densité spatiale, de sorte qu'un élément du second ensemble de données d'entraînement correspond spatialement à une pluralité d'éléments du premier ensemble de données d'entraînement.

13. Procédé de cartographie de caractéristiques du sol, le procédé étant mis en œuvre par un processeur et comprenant :
la cartographie d'au moins un emplacement dans une zone d'intérêt (102) à au moins une valeur prédite d'au moins une caractéristique du sol par un modèle de cartographie du sol, l'au moins une valeur prédite sur la base d'un ensemble d'au moins des premiers et seconds paramètres raffinés pour le modèle de cartographie du sol entraîné selon l'une quelconque des revendications 1 à 12.

14. Système informatique comprenant :
un ou plusieurs processeurs ; et
une mémoire stockant des instructions qui amènent les un ou plusieurs processeurs à effectuer des opérations comprenant le procédé selon l'une quelconque des revendications 1 à 13.
